# EUROPEAN PATENT SPECIFICATION

(11) **EP 1 474 998 B1**
(45) Date of publication and mention of the grant of the patent: **05.12.2007**
(21) Application number: 04009498.9
(22) Date of filing: 22.04.2004
(51) Int. Cl.: A23L 1/29, A23L 1/30, A61K 31/201

(54) **Composition useful for weight control**
Zusammensetzung zur Gewichtskontrolle
Composition pour le contrôle du poids

(30) Priority: 05.05.2003 IT MI20030903
(43) Date of publication of application: 10.11.2004
(73) Proprietor: Dietetic S.P.A, 20090 Caleppio di Settala (MI) (IT)
(72) Inventor: Mezzina, Cosmo, 20090 Caleppio di Settala (Milano) (IT)
(74) Representative: Minoja, Fabrizio

(56) References cited:
- EP-A- 1 175 901
- WO-A-02/43662
- GB-A- 2 353 471
- DATABASE FSTA [Online] INTERNATIONAL FOOD INFORMATION SERVICE (IFIS), FRANkFURT-MAIN, DE; VOORRIPS L E ET AL: "Intake of conjugated linoleic acid, fat, and other fatty acids in relation to postmenopausal breast cancer: the Netherlands Cohort Study on Diet and Cancer." XP002288989 Database accession no. 2003-00-a0457 & AMERICAN JOURNAL OF CLINICAL NUTRITION, vol. 76, no. 4, 2002, pages 873-882, DEP. OF NUTR. EPIDEMIOLOGY, TNO NUTR. & FOOD RES., PO BOX 360, 3700 AJ ZEIST, NETHERLANDS. E-MAIL VOORRIPS(A)VOEDING.TNO.NL

## Description

The present invention relates to nutraceutical compositions containing conjugated linoleic acid and calcium glycerophosphate, useful for body weight control.

### Background of the invention

A number of slimming products containing various active ingredients, such as chitosan, lypase and amylase inhibitors, vegetable extracts (Garcinia cambogia, Citrus aurantium, Gingko biloba, Commiphora mukul), caffeine, carnitine, chromium picolinate, seaweeds, pectins, vegetable fibers, etc. have been available for some time.

The efficacy and safety of some of these products are disputed and in any case there is still the need for new formulations able to provide a satisfactory solution to the problem of the body weight reduction and control.

Not long ago, positive effects of conjugated linoleic acid on lipids metabolism, in particular its ability of increasing the fat-to-fat-free mass ratio, have been described. A clinical study has evidenced that conjugated linoleic acid induces body weight loss in overweight patients (J. Nutr., 130:2943-2948, 2000).

Recently, the role of calcium in the regulation of the lipids metabolism and obesity pathogenesis has also been studied (J. Am. College Nutr., 21(2)146S-151S, 2002; ibidem, 20(5), 428S-435S, 2001; J. Clin. Endocrinol. Metabolism, 85(12), 4635, 2000).

The patent application GB 2353471 discloses compositions for treating obesity comprising a source of claim calcium and a source of short chain fatty acids.

### Disclosure of the invention

It has now been found that the association of calcium glycerophosphate and conjugated linoleic acid (in the following referred to as CLA) allows to obtain surprising and advantageous synergistic effects in the reduction of body weight and fat-to-fat-free mass ratio.

Accordingly, in a first aspect the invention provides nutraceutical compositions containing said association in combination with suitable excipients and optionally other active ingredients having any auxiliary or useful activity.

A further aspect of the invention consists in the use of conjugated linoleic acid and calcium glycerophosphate for the preparation of functional foodstuffs, food supplements or nutraceuticals for the reduction of body weight and increase of fat-free mass.

The compositions of the invention are typically in the form of tablets, capsules, drinkable solutions or suspensions, snaks and the like and contain 50 to 500 mg of CLA and 100 to 500 mg of calcium salt per dose unit, preferably 100 to 300 mg of CLA and 200 to 400 mg of calcium glycerophosphate.

According to a particularly preferred embodiment of the invention, the compositions also contain chromium ions, preferably organic chromium, and ascorbic acid, in amount ranging from 10 to 30 mg and 10 to 100 mg respectively per dose unit.

CLA and calcium glycerophosphate are commercially available.

The compositions of invention, administered usually from one to three times a day, preferably before meals, induce a weight loss significantly higher than that obtainable when CLA and calcium salts are administered separately, and also a significant decrease in the fat-to-fat-free mass ratio.

The invention is illustrated in greater detail by means of the following example.

### EXAMPLE

| **800 mg tablets** | |
|---|---|
| Calcium glycerophosphate | 350 mg |
| CLA | 200 mg |
| Chromium yeast | 25 mg |
| Vitamin C | 15 mg |
| Cellulose microcystalline | 147,80 mg |
| Dibasic calcium phosphate | 22 mg |
| Vanilline | 4 mg |
| Micronised leucine | 8 mg |
| Vegetable magnesium stearate | 5 mg |
| Grapefruit seeds dry extract | 0,20 mg |
| Fatty acids mono and diglycerides | 15 mg |
| Silicon dioxide | 8 mg |
| Total | 800 mg |

## Claims

1. Nutraceutical compositions containing conjugated linoleic acid and calcium glycerophosphate in admixture with suitable excipients.

2. Compositions as claimed in claim 1, in the form of capsules, tablets, drinkable solutions or suspensions, snacks.

3. Compositions as claimed in any one of claims from 1 to 2 further containing chromium and ascorbic acid.

4. Use of conjugated linoleic acid and calcium glycerophosphate for the preparation of functional foodstuffs, food supplements or nutraceuticals for the reduction of body weight and increase of the fat-free mass.

## Patentansprüche

1. Nutrazeutische Zusammensetzungen enthaltend konjugierte Linolsäure und Calciumglycerophosphat in Mischung mit geeigneten Exzipienten.

2. Zusammensetzungen nach Anspruch 1 in der Form von Kapseln, Tabletten, Trinklösungen oder Suspensionen, Zwischenmahlzeiten (Snacks).

3. Zusammensetzungen nach Anspruch 1 oder 2, die weiterhin Chrom und Ascorbinsäure enthalten.

4. Verwendung von konjugierter Linolsäure und Calciumglycerophosaphat für die Herstellung von funktionellen Lebensmitteln, Nahrungsergänzungsmitteln oder Nutrazeutika zur Verringerung des Körpergewichts und zur Erhöhung der fettfreien Masse,

## Revendications

1. Compositions neutraceutiques contenant de l'acide linoléique conjugué et du glycérophosphate de calcium en mélange avec des excipients adaptés.

2. Compositions selon la revendication 1, sous forme de capsules, de comprimés, de solutions ou suspensions buvables, de collations.

3. Compositions selon l'une quelconque des revendications 1 et 2 contenant en outre du chrome et de l'acide ascorbique.

4. Utilisation de l'acide linoléique conjugué et du glycérophosphate de calcium pour la préparation d'aliments fonctionnels, d'aliments complémentaires ou de nutraceutiques pour la réduction du poids corporel et l'augmentation de la masse sans graisse.
